# EUROPEAN PATENT APPLICATION

(11) **EP 0 703 233 A2**
(43) Date of publication of application: **27.03.1996**
(21) Application number: 95111702.7
(22) Date of filing: 25.07.1995
(51) Int. Cl.: C07D 487/02

(54) **Process for the preparation of 5-substituted-1-azabicyclo (3.3.0) octanes**

(30) Priority: 29.07.1994 JP 178507/94
(71) Applicant: SANWA KAGAKU KENKYUSHO CO., LTD., Nagoya, Aichi-ken (JP)
(72) Inventor: Oka, Mitsuru, c/o Sanwa Kagaku Kenkyusho Co., Ltd., Nagoya, Aichi-ken (JP); Suzuki, Tomoo, c/o Sanwa Kagaku Kenkyusho Co.,Ltd., Nagoya, Aichi-ken (JP); Baba, Yutaka, c/o Sanwa Kagaku Kenkyusho Co.,Ltd., Nagoya, Aichi-ken (JP)
(74) Representative: Wächtershäuser, Günter, Prof. Dr.

(57) **Abstract**

There is disclosed a process for the preparation of 5-substituted-1-azabicyclo[3.3.0]octanes of the Formula 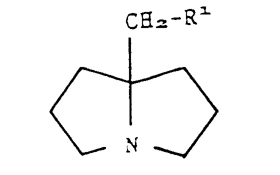
wherein R¹ is a cyano radical or group of -CO₂R³, in which R³ is a lower alkyl group.
The derivative is prepared by reacting 1,7-disubstituted-4-heptanone of the Formula 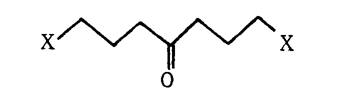
wherein X is a chlorine, bromine or iodine atom, or a group of -OSO₂R, in which R is a methyl, tolyl or tri-fuloromethyl radical,
with a substituted acetic acid derivative of the Formula

R¹CH₂CO₂Y

wherein R¹ has the meaning as referred to; and Y is a hydrogen, sodium or potassium atom, or -NH₄,
an alkali metal salt or ammonium salt of the derivative, in the presence of ammonia. Among the 5-substituted-1-azabicyclo[3.3.0]octanes, 5-cyanomethyl-1-azabicyclo[3.3.0]octane can be converted into 5-(2-aminoethyl)-1-azabicyclo[3.3.0]octane through a catalystic reduction which is carried out in atmospheric pressure, in alkali condition, and in the presence of Raney nickel.

## Description

The invention relates to a novel process for the preparation of 5-substituted-1-azabicyclo[3.3.0]octanes and more particularly, 5-cyanomethyl-1-azabicyclo[3.3.0]octane, 5-alkoxycarbonylmethyl-1-azabicyclo[3.3.0]octane and 5-(2-aminoethyl)-1-azabicyclo[3.3.0]octane.

Each of these compounds has been known per se, is useful as an intermediate for preparing various medicines and agricultural chemicals, since it has an alkaloid skeleton therein, and for instance, can be utilized as a starting material for synthesizing an anti-digestive ulcer [Jap. Pat. Hei 5 (A.D. 1993) - 76948(B)] and an agent for improving cerebral function (EP-A-0 647 642).

According to a conventional process (hereinafter referred to as "Conventional process A"), 5-cyanomethyl-1-azabicyclo[3.3.0]octane has been prepared by reacting Y -butyrolactone with potassium cyanate, heating resulting Y -(N-2-pyrrolidinonyl)butyric acid in the presence of soda lime, reacting resulting 1-azabicyclo[3.3.0]oct-4-ene with perchloric acid, purifying resulting 1-azoniabicyclo[3.3.0]oct-4-ene, reacting the same with potassium hydroxide, and reacting resulting 1-azabicyclo[3.3.0]oct-4-ene with cyanoacetic acid [Miyano et al., "Synthesis", page 701 (1978); and Miyano et al., "Heterocycles", Vol. 16, page 755 (1981). A reaction of sodium salt of 2-pyrrolidone with y -butyrolactone has been known as the process for synthesizing said Y -(N-2-pyrrolidinonyl)butyric acid [Miyano et al., "J. Heterocyclic Chem.", Vol. 19, page 1465 (1982)].

This conventional process A can be shown by following reaction formulae.

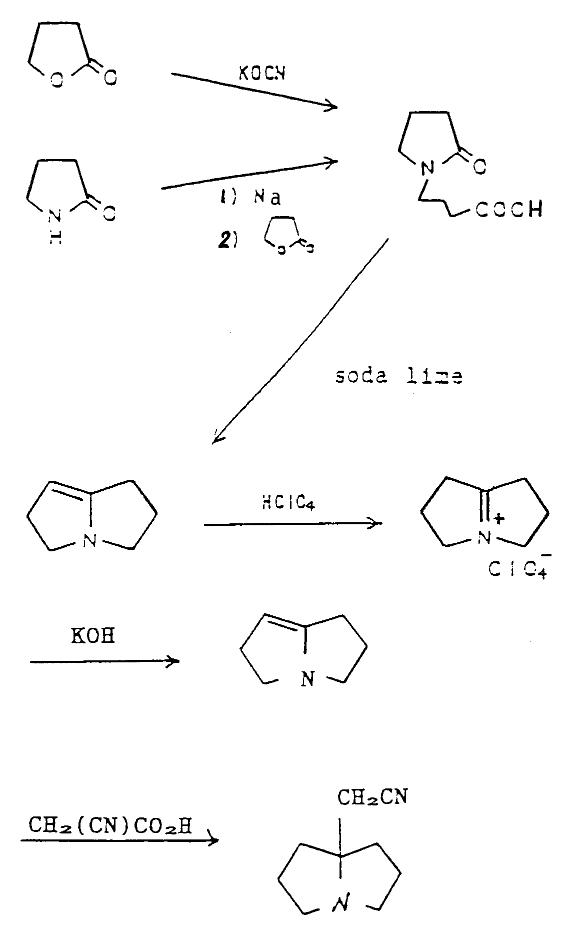

According to another conventional process (hereinafter referred to as "Conventional process B"), 5-cyanomethyl-1-azabicyclo[3.3.0]octane has been prepared by reacting 1,7-disubstituted-4-heptanone with ammonia in the presence of an ammonium salt, and reacting resulting salt of 1-azoniabicyclo[3.3.0]act-1(5)-ene with cyanoacetic acid [Jap. Pat. Hei 5 (A.D. 1993) - 286975(A)].

This conventional process B can be shown by following reaction formulae.

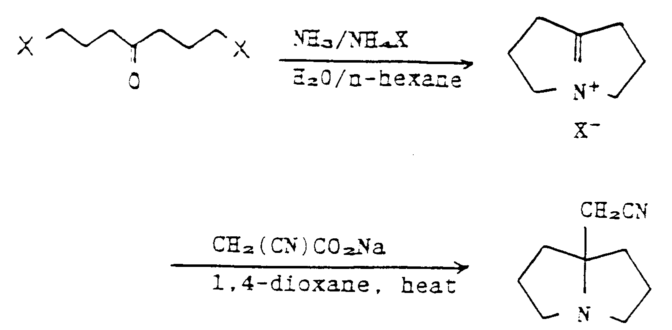

As a conventional process (hereinafter referred to as "Conventional process C) for preparing 5-alkoxycarbonylmethyl-1-azabicyclo[3.3.0]octane, such a process has been known that 5-cyanomethyl-l-azabicyclo[3.3.0]octane derived from a salt of 1-azoniabicyclo[3.3.0]oct-1(5)-ene is reacted with methanol and sulfuric acid to form 5-methoxycarbonylmethyl-1-azabicyclo[3.3.0]octane [Miyano et al., "Heterocycles", Vol. 16, page 755 (1981)].

This conventional process C can be shown by following reaction formulae.

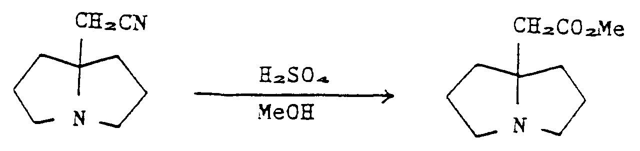

As a conventional process (hereinafter referred to as "Conventional process D) for preparing 5-(2-aminoethyl)-1-azabicyclo[3.3.0]octane, such a process has only been known that 5-cyanomethyl-1-azabicyclo[3.3.0]octane is reduced by lithium aluminum hydride [Miyano et al., "Heterocycles", Vol. 16, page 755 (1981)].

This conventional process D can be shown by following reaction formulae.

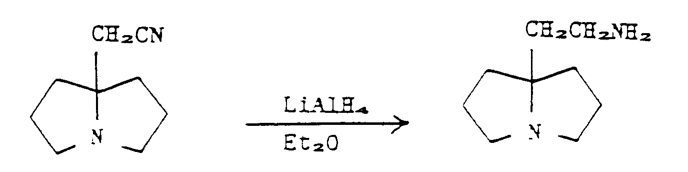

The conventional process A has following disadvantages. Namely, the first step of reacting y -butyrolactone with potassium cyanate to synthesize Y -(N-2-pyrrolidinoyl)butyric acid requires high reaction temperature (about 200°C ) and provides a low yield (about 40%). While, a modified process thereof, namely that of reacting 2-pyrrolidone with metallic sodium and then reacting with y -butyrolactone may cause an abnormal reaction of accidental explosion.

The second step of heating Y -(N-2-pyrrolidinonyl)butyric acid in the presence of soda lime to synthesize 1-azabicyclo[3.3.0]oct-4-ene has also disadvantages of that high reaction temperature (250 - 300°C ) is required and that the reaction product has low stability.

Further, the third step of reacting perchloric acid of 1-azoniabicyclo[3.3.0]oct-1(5)-ene with potassium hydroxide to obtain 1-azabicyclo[3.3.0]oct-4-ene and then reacting the same with cyanoacetic acid to synthesize 5-cyanomethyl-1-azabicyclo[3.3.0]octane has a disadvantage of that stability of the intermediate, 1-azabicyclo[3.3.0]oct-4-ene, is very low.

The conventional process B has following disadvantages. Namely, the first step of reacting 1,7-disubstituted-4-heptanone with ammonia in the presence of the ammonium salt to synthesize the salt of 1-azoniabicyclo[3.3.0]oct-1(5)-ene has such a problem of that the product of 1-azoniabicyclo[3.3.0]oct-1(5)-ene (salt) has low stability in ammonia. Therefore, it is essential to keep low temperature during the reaction and in subsequent procedures of removing ammonia and neutralization of the reaction solution. These become a neck in industrial production. Further, ammonium tetrafluoroborate which is possibly employed in the step is expensive to make difficult an industrial scale production.

The process for the preparation of 5-alkoxycarbonylmethyl-1-azabicyclo[3.3.0]octane according to the conventional process C requires 5-cyanomethyl-1-azabicyclo[3.3.0]octane as the raw material, but there is the problem in the preparation of the raw material per se, as referred to on the conventional process A.

The conventional process D for preparing 5-(2-aminoethyl)-1-azabicyclo[3.3.0]octane has such disadvantages as an industrial process that expensive reduction agent of lithium aluminum hydride is required and that the reaction must be carried out by using anhydrous solvent.

Therefore, the main object of the invention is to provide a process for the preparation of 5-substituted-1-azabicyclo[3.3.0]octanes and more particularly, 5-cyanomethyl-1-azabicyclo[3.3.0]octane and 5-alkoxycarbonylmethyl-1-azabicyclo[3.3.0]octane, which process is easy in operation, has no risk of explosion, does not pass through any unstable intermediate, does not require any expensive reagent and thus is suitable for an industrial scale production of the compounds.

An additional object of the invention is to provide a process advantageous in economy and reaction procedures for converting 5-cyanomethyl-1-azabicyclo[3.3.0]octane into 5-(2-aminoethyl)-1-azabicyclo[3.3.0]octane.

According to the invention, the main object of the invention is attained by a process for the preparation of 5-substituted-1-azabicyclo[3.3.0]octanes of the formula (I) 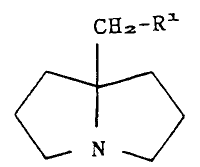
wherein R¹ is a cyano radical or group of -CO₂R, in which R is a lower alkyl group,
which comprises a step of reacting 1,7-disubstituted heptanone of the formula (II) 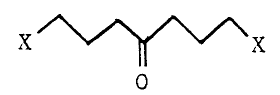
wherein X is a chlorine, bromine or iodine atom, or a group of -OSO₂R³, in which R³ is a methyl, tolyl or trifluoromethyl radical,
with a substituted acetic acid derivative of the formula (III)

R¹CH₂CO₂Y

wherein R¹ has the meaning as referred to; and Y is a hydrogen, sodium or potassium atom, or -NH₄,
an alkali metal salt or ammonium salt of the derivative, in the presence of ammonia.

The additional object is attained by a process for the preparation of 5-(2-aminoethyl)-1-azabicyclo[3.3.0]octane of the formula (IV) 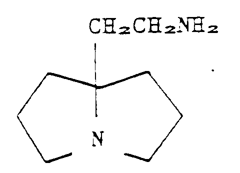
characterized by catalystically reducing 5-cyanomethyl-1-azabicyclo[3.3.0]octane of the formula (V) 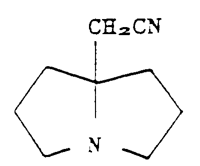
in the presence of Raney nickel, in an atmospheric pressure and in an alkali condition.

For synthesizing the compound shown by Formula (I), in the first place, 2 - 10 equivalents of the compound shown by Formula (III) and 10 - 100 equivalents of ammonia were added to 1 equivalent of the compound (II) to stir for 10 - 60 hours at a temperature of 0 - 30°C in the presence of a solvent. Then, almost all of ammonia was distilled out, a solvent is added, if necessary, to stir for 1 - 5 hours at a temperature not lower than than 50°C , the reaction mixture was made in alkali condition and then extracted by an organic solvent to afford the desired 5-substituted-l-azabicyclo[3.3.0]octane.

Such a solvent can be used for the above operations : water; methanol, ethanol, n-propanol, i-propanol or the like alcohol; ethyl ether, tetrahydrofuran, 1,4-dioxane or the like ether; n-hexane, benzene, toluene or the like organic solvent. As the organic solvent for extraction, ethyl acetate, methylene chloride, chloroform or ethyl ether or the like can be listed.

The raw material of 1,7-disubstituted-4-heptanone can easily be synthesized from y -butyrolactone, in accordance with a method disclosed by H. Hart et al. [J. Am. Chem. Soc.", Vol. 78, page 112 (1956)].

For synthesizing the compound shown by Formula (IV), 0.1 - 10 equivalents of the base and 5 - 60% by weight of Raney nickel were added to 1 equivalent of the compound shown by Formula (V). The catalystic reduction can be carried out at a temperature of 0 - 50°C in the atmospheric pressure and in the presence of a solvent to afford the desired 5-(2-aminoethyl)-1-azabicyclo[3.3.0]octane. Although it has generally been said that high-temperature and high-pressure conditions are essential for catalystically reducing cyano radical in the presence of Raney nickel catalyst ["J. Am. Chem. Soc.", Vol. 68, page 2499 (1946)], the desired 5-(2-aminoethyl)-1-azabicyclo[3.3.0]octane shown by Formula (IV) can unexpectedly be obtained with good yield, through the catalystic reduction under said conditions and more preferably, adding 2 equivalents of sodium hydroxide as the base and 40% by weight of Raney nickel to 1 equivalent of the compound shown by Formula (IV), and conducting the catalystic reduction in methanol as the solvent.

In the above procedure, ammonia, sodium hydroxide, potassium hydroxide or the like can be used as the base, and water or an alcohol such as methanol, ethanol, n-propanol and i-propanol can be used as the solvent.

The invention will now be further explained in more detail with reference to Examples for preparing compounds.

### Example 1 (5-Cyanomethyl-1-azabicyclo[3.3.0]octane)

To 1.9kg of ammonia in 2.4kg of distilled water, were added 2.67kg of cyanoacetic acid, 1.15kg of 1,7-dichloro-4-heptanone and 1.15 liters of hexane to stir for 40 hours at a temperature of 15 - 20°C . After removed hexane layer from the reaction mixture and then distilled out almost all of ammonia in vacuo, the reaction mixture was stirred for 1 hour at 70°C . The reaction solution was made into alkaline one and extracted by ethyl acetate. The organic layer was dried over anhydrous sodium sulfate and concentrated to afford 551g of the titled compound (Yield : 57.5%).

Boiling point : 82 - 83°C (8mmHg),

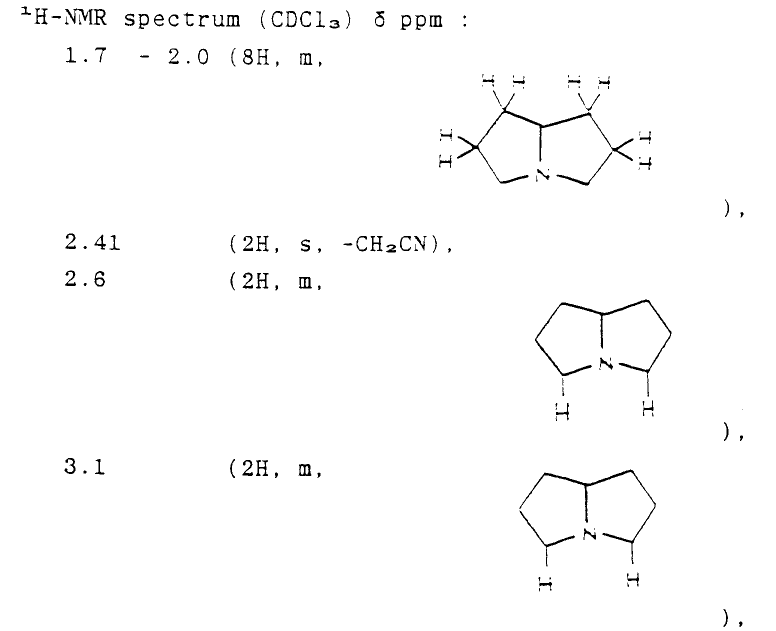

### Example 2 (5-Ethoxycarbonylmethyl-1-azabicyclo[3.3.0]octane)

To 1ml of ethanol, were added 0.10g of 1,7-dichloro-4-heptanone and 0.28g of malonic acid monoethyl ester potassium salt to stir 40 hours at a temperature of 15 - 20°C and under ammonia gas atmosphere. After removed almost all of ammonia from the reaction mixture in vacuo, dioxane was added to stir for 2 hours at 80°C . After concentrated and added water, the reaction mixture was extracted by chloroform, dried the organic layer over anhydrous sodium sulfate and concentrated to afford 45mg of the titled compound (Yield : 40%).

Boiling point : 90°C (8mmHg).

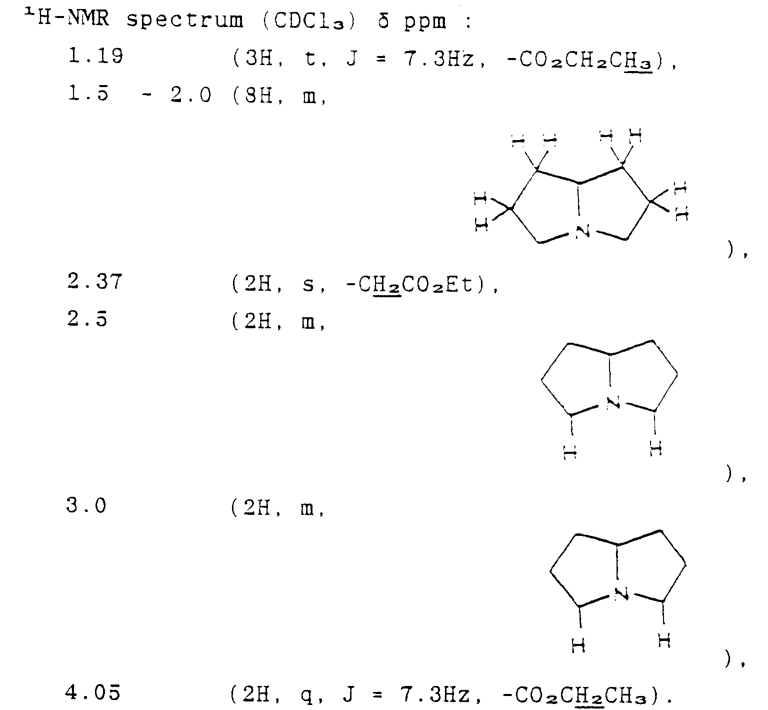

### Example 3 {5-(2-Aminoethyl)-1-azabicyclo[3.3.0]octane}

To 294g of sodium hydroxide in 3.8 liters of methanol, was added 110g of Raney nickel. After added 550g of 5-cyanomethyl-1-azabicyclo[3.3.0]octane obtained by Example 1 thereto to carry out a catalystic reducing reaction for 24 hours at atmospheric pressure. After removed Raney nickel, the reaction mixture was concentrated and added water to the resulting residue to extract by chloroform. The organic layer was dried over anhydrous sodium sulfate and concentrated to afford 507g of the titled compound (Yield : 89.6%).

Boiling point : 82 - 84°C (4mmHg).

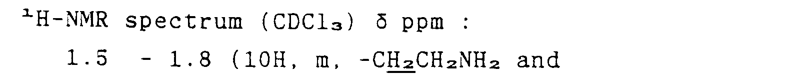

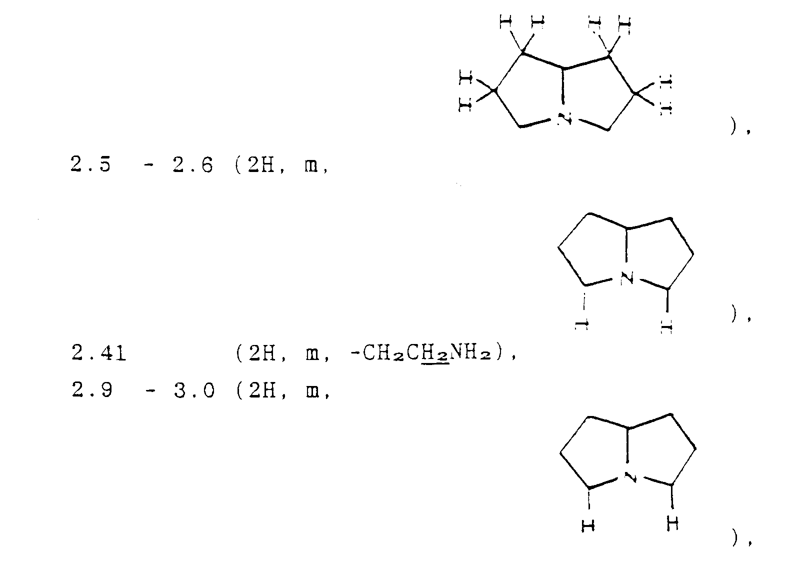

## Claims

1. A process for the preparation of 5-substituted-1-azabicyclo[3.3.0]octanes of the formula (I) 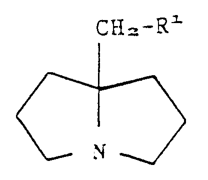 wherein R¹ is a cyano radical or group of -CO₂R, in which R is a lower alkyl group,
which comprises a step of reacting 1,7-disubstituted heptanone of the formula (II) 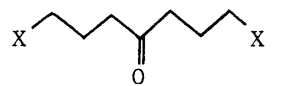 wherein X is a chlorine, bromine or iodine atom, or a group of -OSO₂R³, in which R³ is a methyl, tolyl or trifluoromethyl radical,
with a substituted acetic acid derivative of the formula (III)
R¹CH₂CO₂Y
wherein R¹ has the meaning as referred to; and Y is a hydrogen, sodium or potassium atom, or -NH₄,
an alkali metal salt or ammonium salt of the derivative, in the presence of ammonia.

2. A process for the preparation of 5-(2-aminoethyl)-1-azabicyclo[3.3.0]octane of the formula (IV) 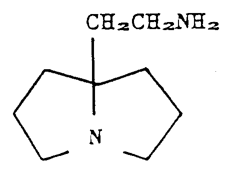 characterized by catalystically reducing 5-cyanomethyl-1-azabicyclo[3.3.0]octane of the formula (V) 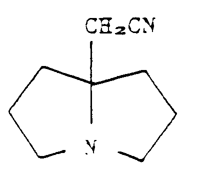 in the presence of Raney nickel, in an atmospheric pressure and in an alkali condition.
